# EUROPÄISCHE PATENTSCHRIFT

(11) **EP 3 643 699 B1**
(45) Veröffentlichungstag und Bekanntmachung des Hinweises auf die Patenterteilung: **22.09.2021**
(21) Anmeldenummer: 19202629.2
(22) Anmeldetag: 11.10.2019
(51) Int. Cl.: C07C 209/84, C07C 211/46

(54) **VERFAHREN ZUR REINIGUNG VON ANILIN**
METHOD FOR THE PURIFICATION OF ANILINE
PROCÉDÉ DE PURIFICATION D'ANILINE

(30) Priorität: 17.10.2018 EP 18200968
(43) Veröffentlichungstag der Anmeldung: 29.04.2020
(73) Patentinhaber: Covestro Intellectual Property GmbH & Co. KG, 51373 Leverkusen (DE)
(72) Erfinder: MERKEL, Michael, 40223 Düsseldorf (DE); STEFFENS, Friedhelm, 51373 Leverkusen (DE); KALEM, Murat, 41462 Neuss (DE); LEHNER, Peter, 45481 Mühlheim an der Ruhr (DE)
(74) Vertreter: Levpat

(56) Entgegenhaltungen:
- EP-A1- 2 028 176
- DE-A1-102004 026 626

## Beschreibung

Die vorliegende Erfindung betrifft ein Verfahren zur Reindarstellung von Anilin, umfassend die Schritte a) Bereitstellen einer Rohanilinfraktion enthaltend, bezogen auf die Gesamtmasse von Anilin und Phenol in der Rohanilinfraktion, 80,000 Massen-% bis 99,999 Massen-% Anilin und 0,001 Massen-% bis 20,000 Massen-% Phenol; b) Aufteilen der Rohanilinfraktion aus Schritt a) in einen ersten Teilstrom und einen zweiten Teilstrom; c) Destillieren des ersten Teilstroms aus Schritt b) in einer ersten Destillationskolonne und Destillieren des zweiten Teilstroms aus Schritt b) in einer zweiten Destillationskolonne, wobei die erste Destillationskolonne bei einem höheren Kopfdruck betrieben wird als die zweite Destillationskolonne unter der Maßgabe, dass die erste Destillationskolonne und die zweite Destillationskolonne jeweils bei einem Kopfdruck im Bereich von 1,0 mbar_{(abs.)} bis 500 mbar_{(abs.)}, bevorzugt im Bereich von 10 mbar_{(abs.)} bis 400 mbar_{(abs.)}, betrieben werden, und wobei der ersten Destillationskolonne und der zweiten Destillationskolonne jeweils (i) ein Anilin-Destillat (= gereinigtes Anilin) gasförmig als Kopfprodukt entnommen und anschließend kondensiert wird, wobei ein erster Teilstrom des kondensierten Anilin-Destillats als Rücklaufstrom in die erste Destillationskolonne und/oder in die zweite Destillationskolonne zurückgeführt und ein zweiter Teilstrom des kondensierten Anilin-Destillats als Produktstrom abgeführt wird, und (ii) ein an Phenol angereicherter flüssiger Sumpfstrom entnommen wird, von dem ein erster Teil partiell oder vollständig verdampft und in die jeweilige Destillationskolonne zurückgeführt wird, und von dem ein zweiter Teil ausgeschleust wird; d) Verwenden der bei der Kondensation des gasförmigen Anilin-Destillats aus der ersten Destillationskolonne freiwerdenden Wärme zur Bereitstellung der zur Verdampfung des ersten Teils des Sumpfstromes der zweiten Destillationskolonne erforderlichen Wärme.

Anilin ist ein wichtiges Zwischenprodukt z. B. zur Herstellung der Di- und Polyisocyanate der Diphenylmethanreihe (MDI) und wird im großtechnischen Maßstab in der Regel durch katalytische Hydrierung von Nitrobenzol in der Gas- oder Flüssigphase hergestellt (siehe z.B. DE-OS 2201528, DE-OS 3414714, US-A-3136818, EP-A-0696573 und EP-A-0696574). Bei dieser Reaktion werden neben dem Zielprodukt Anilin auch Nebenkomponenten wie z. B. Phenol oder Aminophenole gebildet, die vor einer weiteren Anwendung des Anilins in Folgeprozessen entfernt werden müssen. Besonders problematisch ist die Abtrennung von solchen Nebenkomponenten, deren Siedepunkte denen des herzustellenden Amins sehr ähnlich sind, weil hier der destillative Aufwand erheblich ist. Im Fall der Herstellung von Anilin (Siedepunkt bei Normaldruck 184 °C) stellt insbesondere die Abtrennung von Phenol (Siedepunkt bei Normaldruck 182 °C) eine große Herausforderung für die Destillationstechnik dar, was sich in der Verwendung langer Destillationskolonnen mit großer Trennstufenzahl und hohen Rücklaufverhältnissen und entsprechend hohem Energieaufwand widerspiegelt. Eine Vakuumdestillation zur Entfernung von Phenol aus Anilin ist z. B. in DE1935363 beschrieben.

Aus diesem Grund hat es nicht an Ansätzen gemangelt, Phenol (und anderer phenolische Verbindungen) auf andere Art von Anilin zu trennen, insbesondere durch Überführung in Phenolatsalze durch Reaktion mit geeigneten Basen. Die dabei gebildeten Phenolatsalze lassen sich als nichtflüchtige, gut wasserlösliche Verbindungen wesentlich leichter von Anilin trennen als die phenolischen Verbindungen selbst.

So wird in JP-A-49-035341 ein Verfahren beschrieben, bei dem Anilin mit festen Alkalihydroxiden in einem Festbett in Kontakt gebracht und erst anschließend in die Destillation geleitet wird, bzw. bei dem die Destillation in Gegenwart des festen Alkalihydroxides in Anteilen von 0,1 bis 3 Massen-%, bezogen auf die zu destillierende Anilinmenge, durchgeführt wird. Hierdurch wird die Abtrennung kritischer Komponenten wie der Aminophenole vereinfacht. Nachteilig bei diesem Verfahren ist jedoch der Einsatz von hohen molaren Überschüssen der festen Alkalihydroxide in Bezug auf die zu entfernenden sauren Nebenkomponenten und die Unmöglichkeit der genauen Dosierung der alkalischen Verbindungen. Dies kann einerseits bei Überdosierung zu Korrosionsproblemen, Ausfällungen und hochviskosen Sumpfphasen in der Destillationskolonne und andererseits bei Unterdosierung zu einer unvollständigen Entfernung der kritischen Komponenten führen.

US-A-2005 080294 beschreibt ein Verfahren zur Abtrennung von Verbindungen mit phenolischen Hydroxygruppen von aromatischen Aminen, wobei dem zu reinigenden Amin vor der Destillation eine Base im molaren Verhältnis von 1 : 1 bis zu 4 : 1 bezogen auf die phenolischen Verbindungen, zugesetzt wird, optional in Gegenwart von Polyolen, wie z. B. Polyethylenglykol. Gerade für Anilin, welches in sehr großen Mengen hergestellt wird, ist eine solche Zugabe von Polyolen jedoch wirtschaftlich nachteilig und es besteht die Gefahr, das Produkt mit Polyol(-fragmenten) zu kontaminieren. Ohne Einsatz eines solchen Polyols ist aber mit häufigen Prozessunterbrechungen durch Ausfällung von Salzen zu rechnen.

EP-A-1 845 079 beschreibt ein Verfahren zur Reinigung von Anilin durch Zugabe einer wässrigen Alkalimetallhydroxid-Lösung vor oder während der Destillation, wobei die Probleme durch Feststoffabscheidung, Fouling und/oder starken Viskositätsanstieg bei der Destillation dadurch verhindert werden, dass die Sumpfphase der Destillation partiell ausgeschleust, mit Wasser oder verdünnter Alkalihydroxidlösung gewaschen und die organische gewaschene Phase wieder in die Destillation zurückgeführt wird. Nachteilig ist hier die Notwendigkeit eines zusätzlichen Verfahrensschrittes zur Aufrechterhaltung eines zuverlässigen Betriebs. Darüber hinaus fällt in diesem Verfahren ein zusätzlicher, organisch belasteter Abwasserstrom an, der aufbereitet und entsorgt werden muss.

EP-A-2 028 176 beschreibt ein Verfahren zur Reinigung von aromatischen Aminen, bei dem das nach Abtrennung des Prozesswassers erhaltene rohe Amin mit wässriger Alkalimetallhydroxid-Lösung versetzt und das so erhaltene Verfahrensprodukt destilliert wird. Der Sumpf der Destillationskolonne wird teilweise bis vollständig ausgeschleust und teilweise über zwei seriell oder parallel geschaltete Verdampfer (E1) und (E2) verdampft. Hierdurch soll mit minimalem apparativem und energetischem Aufwand eine maximale Abreicherung des wertvollen Amins im Sumpf der Destillationskolonne erreicht werden.

Bei allen bisher genannten Verfahren wird das aromatische Amin in Gegenwart einer Base destilliert. Bei dieser Verfahrensweise müssen Probleme durch Korrosion, Feststoffabscheidung und/oder Fouling bei der Destillation durch aufwändige und/oder teure Maßnahmen verhindert werden.

Daneben gibt es solche Verfahren, die das Phenol durch eine Extraktion mit wässriger Base entfernen. Um auch andere, nicht acide Verunreinigungen zu entfernen, muss das Anilin dennoch zumindest teilweise destilliert werden. Solche Verfahren sind beispielsweise in DE102009025374, JP-A-08-295654 oder auch in EP 2 641 892 A1 beschrieben. Um heutige Reinheitsanforderungen zu erfüllen, werden in der Regel mehrere Extraktionsstufen benötigt wie z. B. in JP- A-2007217405 beschrieben, was den Aufwand weiter erhöht. Außerdem fallen in allen diesen Verfahren große Phenolat-haltige Abwasserströme an, die wiederum sorgfältig aufbereitet und entsorgt werden müssen.

Schließlich ist in DE 10 2004 026626 ein Verfahren beschrieben, bei dem wässrige Amin-Lösung in zwei in Serie geschalteten Destillationskolonnen aufgereinigt wird. Dabei wird eine der Kolonnen bei 2 bis 20 bar und die andere bei 0,1 bis 10 bar betrieben. Die Kondensationswärme der Brüden, die aus der mit höherem Druck betriebenen Kolonne austreten, werden dabei verwendet, um den Sumpf der mit niedrigerem Druck betriebenen Kolonne zu beheizen. Das Amin fällt als Sumpfprodukt an. Deshalb eignet sich das Verfahren nicht zur Abtrennung von Hochsiedern oder Phenol aus Anilin. Aufgrund des hohen Drucks sind außerdem hohe Sumpftemperaturen erforderlich, was zu einer teilweisen Zersetzung des Produkts und damit zu Ausbeuteverlusten oder Fouling führen kann.

Es bestand daher ein Bedarf an weiteren Verbesserungen auf dem Gebiet der Anilinreinigung. Insbesondere war es wünschenswert, die Abtrennung von gegenüber Anilin hochsiedenden Verbindungen, insbesondere Phenol, auf effiziente, energiesparende Art zu erreichen. Auch war es wünschenswert, eine Basenbehandlung des Anilins möglichst überflüssig zu machen.

Dem vorstehend Gesagten Rechnung tragend ist ein Gegenstand der vorliegenden Erfindung ein Verfahren zur Reindarstellung von Anilin, umfassend die folgenden Schritte:
a) Bereitstellen einer Rohanilinfraktion enthaltend, bezogen auf die Gesamtmasse von Anilin und Phenol in der Rohanilinfraktion, 80,000 Massen-% bis 99,999 Massen-% Anilin und 0,001 Massen-% bis 20,000 Massen-% Phenol, insbesondere enthaltend, bezogen auf die Gesamtmasse von Anilin und Phenol in der Rohanilinfraktion, 95,000 Massen-% bis 99,995 Massen-% Anilin und 0,005 Massen-% bis 5,000 Massen-% Phenol, bevorzugt 98,000 Massen-% bis 99,990 Massen-% Anilin und 0,010 Massen-% bis 2,000 Massen-% Phenol, besonders bevorzugt 99,000 Massen-% bis 99,990 Massen-% Anilin und 0,010 Massen-% bis 1,000 Massen-% Phenol, ganz besonders bevorzugt 99,900 Massen-% bis 99,990 Massen-% Anilin und 0,010 Massen-% bis 0,100 Massen-% Phenol, wobei von Phenol verschiedene organische Verbindungen (die als weitere Verunreinigungen vorhanden sein können, insbesondere Benzol, Cyclohexylamin, Cyclohexanon, Cyclohexanol Aminophenole, Diphenylamin, N-Cyclohexylanilin und Phenylendiamin) bevorzugt in einem auf die Gesamtmasse des Rohanilinstroms bezogenen Anteil von 2,00 Massen-%, besonders bevorzugt maximal 1,00 Massen-%, besonders bevorzugt maximal 0,50 Massen-%, vorhanden sind;
b) Aufteilen der Rohanilinfraktion aus Schritt a) in Teilströme, und zwar mindestens in einen ersten Teilstrom und einen zweiten Teilstrom;
c) Destillieren des ersten Teilstroms aus Schritt b) in einer ersten Destillationskolonne und Destillieren des zweiten Teilstroms aus Schritt b) in einer zweiten Destillationskolonne, wobei die erste Destillationskolonne bei einem höheren Kopfdruck betrieben wird als die zweite Destillationskolonne unter der Maßgabe, dass die erste Destillationskolonne und die zweite Destillationskolonne jeweils bei einem Kopfdruck im Bereich von 1,0 mbar_{(abs.)} bis 500 mbar_{(abs.)}, bevorzugt im Bereich von 10 mbar_{(abs.)} bis 400 mbar_{(abs.)}, besonders bevorzugt im Bereich von 50 mbar_{(abs.)} bis 350 mbar_{(abs.)}, betrieben werden, und wobei der ersten Destillationskolonne und der zweiten Destillationskolonne jeweils
   - ein Anilin-Destillat gasförmig als Kopfprodukt entnommen und anschließend kondensiert wird, wobei ein erster Teilstrom des kondensierten Anilin-Destillats als Rücklaufstrom in die erste Destillationskolonne und/oder in die zweite Destillationskolonne zurückgeführt und ein zweiter Teilstrom des kondensierten Anilin-Destillats als Produktstrom abgeführt wird, und
   - ein an Phenol angereicherter flüssiger Sumpfstrom entnommen wird, von dem ein erster Teil partiell oder vollständig verdampft und in die jeweilige Destillationskolonne zurückgeführt wird (im Fall der *partiellen* Verdampfung können erhaltener Dampf und verbleibende Flüssigkeit getrennt oder gemeinsam zurückgeführt werden), und von dem ein zweiter Teil ausgeschleust wird;
d) Verwenden der bei der Kondensation des Anilin-Destillats aus der ersten Destillationskolonne freiwerdenden Wärme zur Bereitstellung der zur Verdampfung des ersten Teils des Sumpfstromes der zweiten Destillationskolonne erforderlichen Wärme.

*Gehaltsangaben organischer Verbindungen* beziehen sich im Rahmen der vorliegenden Erfindung, sofern nicht anders angegeben, auf per Gaschromatographie bestimmte Werte. Die quantitative Auswertung von Gaschromatogrammen ist dem Fachmann bekannt. Gegebenenfalls erforderliche Methoden zur Bestimmung des *Wassergehalts* sind dem Fachmann ebenfalls bekannt. Die Methoden des Standes der Technik können auch im Rahmen der vorliegenden Erfindung angewandt werden. Im Zweifelsfall ist der per Karl-Fischer-Titration bestimmte Wassergehalt maßgeblich. Zur Karl-Fischer-Titration allgemein, siehe Jander, Jahr, Maßanalyse, 17. Aufl., de Gruyter, Berlin (2009), S. 279 bis S. 282). Bei der Bestimmung des Wassergehalts von Anilinfraktionen (insbesondere der in Schritt a) bereitzustellenden Rohanilinfraktion) ist der per Karl-Fischer-Titration nach Pufferung mit Salicylsäure bestimmte Wert maßgeblich. Das Verfahren unter Pufferung mit Salicylsäure ist dem Fachmann ebenfalls bekannt; siehe beispielsweise Merck KGaA, Wasser in Anilin - Karl Fischer Applikation, 2011 und Honeywell, HYDRANAL™ Praktikum - Reagenzien nach Eugen Scholz für die Karl-Fischer-Titration, 2017).

Hier und im Folgenden sind alle *Drücke* als absolute Drücke (gekennzeichnet als "bar_{(abs.)}") zu verstehen.

Ausdrücke wie *"ein erster Teilstrom und ein zweiter Teilstrom"* oder *"eine erste Destillationskolonne und eine zweite Destillationskolonne"* oder "*ein erster Teil und ein zweiter Teil"* sind, sofern nicht ausdrücklich anders gesagt, stets als offene Formulierungen aufzufassen, die das Vorhandensein weiterer (dritter, vierter, ...) Teilströme, Destillationskolonnen oder Teile nicht ausschließen.

Der Ausdruck *"Rücklaufstrom"* oder kurz *"Rücklauf"* bezeichnet den Teil des kondensierten Kopfproduktes einer Destillationskolonne, der nicht als Produktstrom abgeführt, sondern in die Destillationskolonne (oder gegebenenfalls in eine andere Destillationskolonne) zurückgeführt wird. Der Ausdruck *"Rücklaufverhältnis"* bezieht sich im Rahmen dieser Beschreibung auf das Massenverhältnis des Rücklaufstroms zum abgeführten Strom (Produktstrom). Das Rücklaufverhältnis wird auch als *"R*/*E-Verhältnis "* bezeichnet.

Überraschend wurde gefunden, dass die Probleme des Standes der Technik gelöst oder zumindest vermindert werden können, wenn Rohanilin, vorzugsweise getrocknetes Rohanilin (d. h. Rohanilin, dessen auf die Gesamtmasse des Rohanilins bezogener Anteil an Wasser im Bereich von 0,00 Massen-% bis 0,50 Massen-%, bevorzugt 0,00 Massen-% bis 0,20 Massen-%, besonders bevorzugt 0,00 Massen-% bis 0,10 Massen-% und ganz besonders bevorzugt 0,00 Massen-% bis 0,05 Massen-%, insbesondere 0,01 Massen-% bis 0,05 Massen %, liegt), in mindestens zwei parallel betriebenen Destillationskolonnen mit unterschiedlichem Kopfdruck destilliert wird, wobei in keiner der eingesetzten Destillationskolonnen ein Kopfdruck von 500 mbar_{(abs.)}, bevorzugt 400 mbar_{(abs.)}, besonders bevorzugt 350 mbar_{(abs.)}, überschritten wird (und ein Kopfdruck von 1,0 mbar_{(abs.)}, bevorzugt 10 mbar_{(abs.)}, besonders bevorzugt 50 mbar_{(abs.)}, nicht unterschritten wird), und wobei mindestens zwei der Destillationskolonnen wärmetechnisch gekoppelt sind, indem die Wärme aus der Brüdenkondensation der Destillationskolonne mit höherem Kopfdruck direkt oder indirekt zur Beheizung des Sumpfstromes der Destillationskolonne mit niedrigerem Kopfdruck genutzt wird. Dies ermöglicht eine effiziente Abtrennung von Phenol und gegebenenfalls anderen hoch siedenden Nebenkomponenten.

In den beigefügten Zeichnungen zeigen
- **FIG. 1**: eine Destillationsanordnung des Standes der Technik zur Reinigung von Rohanilin;
- **FIG. 2**: eine für die Durchführung der Schritte c) und d) des erfindungsgemäßen Verfahrens geeignete Destillationsanordnung.

Es folgt zunächst eine Kurzzusammenfassung verschiedener möglicher Ausführungsformen.

In einer **ersten Ausführungsform** der Erfindung, die mit allen anderen Ausführungsformen kombiniert werden kann, umfasst das Bereitstellen der Rohanilinfraktion in Schritt a) die Destillation einer neben Phenol zusätzlich Wasser und Leichtsieder enthaltenden Anilinfraktion, wobei die in Schritt a) bereitzustellende Rohanilinfraktion als Sumpfprodukt dieser Destillation erhalten wird und, bezogen auf ihre Gesamtmasse, maximal 0,50 Massen-% Wasser, bevorzugt maximal 0,20 Massen-% Wasser, besonders bevorzugt maximal 0,10 Massen-% Wasser und ganz bevorzugt 0,05 Massen-% Wasser enthält. Bezogen auf ihre Gesamtmasse enthält diese Rohanilinfraktion bevorzugt maximal 0,100 Massen-% Benzol, besonders bevorzugt maximal 0,010 Massen-% Benzol und ganz besonders bevorzugt maximal 0,001 Massen-% Benzol.

In einer **zweiten Ausführungsform** der Erfindung, die eine besondere Ausgestaltung der ersten Ausführungsform ist, wird die neben Phenol zusätzlich Wasser und Leichtsieder enthaltende Anilinfraktion durch katalytische Hydrierung von Nitrobenzol, gefolgt von einer Phasentrennung in eine organische und eine wässrige Phase, als organische Phase erhalten, wobei insbesondere die als Sumpfprodukt der Destillation dieser organischen Phase erhaltene Rohanilinfraktion, bezogen auf ihre Gesamtmasse, maximal 0,300 Massen-% gegenüber Anilin leichter siedende organische Verbindungen (sog. Leichtsieder), bevorzugt maximal 0,200 Massen-% Leichtsieder und besonders bevorzugt maximal 0,100 Massen-% Leichtsieder enthält.

In einer **dritten Ausführungsform** der Erfindung, die mit allen anderen Ausführungsformen kombiniert werden kann, wird die Rohanilinfraktion in Schritt b) in genau zwei Teilströme aufgeteilt, welche in Schritt c) in genau zwei Destillationskolonnen destilliert werden.

In einer **vierten Ausführungsform** der Erfindung, die eine besondere Ausgestaltung der dritten Ausführungsform ist, wird die zweite Destillationskolonne bei einem Kopfdruck im Bereich von 50 mbar_{(abs.)} bis 300 mbar_{(abs.)}, bevorzugt im Bereich von 80 mbar_{(abs.)} bis 200 mbar_{(abs.)}, besonders bevorzugt im Bereich von 100 mbar_{(abs.)} bis 150 mbar_{(abs.)} betrieben.

In einer **fünften Ausführungsform** der Erfindung, die eine besondere Ausgestaltung der dritten und vierten Ausführungsform ist, wird die erste Destillationskolonne bei einem Kopfdruck im Bereich von 250 mbar_{(abs.)} bis 500 mbar_{(abs.)}, bevorzugt im Bereich von 270 mbar_{(abs.)} bis 400 mbar_{(abs.)}, besonders bevorzugt im Bereich von 280 mbar_{(abs.)} bis 350 mbar_{(abs.)} betrieben.

In einer **sechsten Ausführungsform** der Erfindung, die eine besondere Ausgestaltung der dritten, vierten und fünften Ausführungsform ist, wird die erste Destillationskolonne bei einem Kopfdruck betrieben, der um 80 mbar_{(abs.)} bis 300 mbar_{(abs.)}, bevorzugt um 100 mbar_{(abs.)} bis 200 mbar_{(abs.)}, besonders bevorzugt um 110 mbar_{(abs.)} bis 170 mbar_{(abs.)}, höher ist als der Kopfdruck der zweiten Destillationskolonne.

In einer **siebten Ausführungsform** der Erfindung, die eine besondere Ausgestaltung der dritten, vierten, fünften und sechsten Ausführungsform ist, werden mindestens ein Teil der Kondensation des Anilin-Destillats aus der ersten Destillationskolonne und die Verdampfung des ersten Teils des Sumpfstromes der ersten Destillationskolonne in ein und demselben Wärmeaustauscher durchgeführt.

In einer **achten Ausführungsform** der Erfindung, die eine besondere Ausgestaltung der dritten, vierten, fünften, sechsten und siebten Ausführungsform ist, beträgt der Anteil der in Schritt a) bereitgestellten Rohanilinfraktion, welcher in der ersten Destillationskolonne destilliert wird, 5,00 % bis 49,00 %, bevorzugt 15,00 % bis 45,00 %, besonders bevorzugt 25,00 % bis 40,00 % der in Schritt a) bereitgestellten Rohanilinfraktion, wobei der verbleibende Anteil in der zweiten Destillationskolonne destilliert wird.

In einer **neunten Ausführungsform** der Erfindung, die eine besondere Ausgestaltung der dritten, vierten, fünften, sechsten, siebten und achten Ausführungsform ist, ist das Verhältnis von Rücklaufstrom zu Produktstrom in der zweiten Destillationskolonne geringer als in der ersten Destillationskolonne.

In einer **zehnten Ausführungsform** der Erfindung, die eine besondere Ausgestaltung der dritten, vierten, fünften, sechsten, siebten, achten und neunten Ausführungsform ist, wird ein Teil des der ersten Destillationskolonne gasförmig entnommenen Anilin-Destillats gemeinsam mit dem der zweiten Destillationskolonne gasförmig entnommenen Anilin-Destillat in einem Kondensator kondensiert.

In einer **elften Ausführungsform** der Erfindung, die mit allen anderen Ausführungsformen kombiniert werden kann, umfasst das Verfahren den weiteren Schritt:
e) Verwenden der bei der Kondensation des der zweiten Destillationskolonne gasförmig entnommenen Anilin-Destillats anfallenden Wärme zur Erzeugung von Dampf und/oder zur Beheizung einer Trennkolonne, in welcher ein Anilin-Wasser-Azeotrop aus einem Anilin-haltigen Abwasser entfernt wird.

In einer **zwölften Ausführungsform** der Erfindung, die eine besondere Ausgestaltung der elften Ausführungsform ist, wird die Trennkolonne bei einem Kopfdruck im Bereich von 0,80 bar_{(abs.)} bis 1,20 bar_{(abs.)}, bevorzugt im Bereich von 1,00 bar_{(abs.)} bis 1,10 bar_{(abs.)}, betrieben.

In einer **dreizehnten Ausführungsform** der Erfindung, die mit allen anderen Ausführungsformen kombiniert werden kann, enthält die in Schritt a) bereitgestellte Rohanilinfraktion, bezogen auf die Gesamtmasse von Anilin und Phenol in der Rohanilinfraktion, 95,000 Massen-% bis 99,995 Massen-% Anilin und 0,005 Massen-% bis 5,000 Massen-% Phenol.

In einer **vierzehnten Ausführungsform** der Erfindung, die mit allen anderen Ausführungsformen kombiniert werden kann, wird bei jeder Destillationskolonne der erste Teilstrom des kondensierten Anilin-Destillats als Rücklaufstrom in diejenige Destillationskolonne zurückgeführt, welcher das Anilin-Destillat entnommen wurde (d. h. der Rücklauf wird in die *Ausgangs-Destillationskolonne* zurückgeführt).

Die zuvor kurz geschilderten Ausführungsformen und weitere mögliche Ausgestaltungen der Erfindung werden im Folgenden näher erläutert. Dabei können die Ausführungsformen beliebig miteinander kombiniert werden, sofern sich aus dem Zusammenhang nichts anderes ergibt.

Die im Stand der Technik derzeit üblichen industriellen Verfahren zur Herstellung von Anilin (insbesondere die Hydrierung von Nitrobenzol, in geringerem Umfang die Reduktion von Nitrobenzol mit unedlen Metallen wie Eisen, sowie - heute nur noch wenig bedeutsam - die Ammonolyse von Phenol oder Chlorbenzol) liefern trotz ihrer Verschiedenheit letztlich alle ein Phenol-haltiges Rohanilin, sodass das erfindungsgemäße Verfahren auf alle diese Herstellverfahren sinnvoll anwendbar ist und somit auch das in Schritt a) bereitzustellende Rohanilin aus allen diesen Verfahren stammen kann.

Vor einer im Stand der Technik regelmäßig durchgeführten Trocknung enthält dieses im Allgemeinen neben Phenol (und gegebenenfalls anderen Hochsiedern) mindestens noch Leichtsieder und Wasser. Unter "*Leichtsiedern*" werden im Rahmen der vorliegenden Erfindung solche organischen Verbindungen (und gegebenenfalls Azeotrope) verstanden, die unter den für eine Destillation gewählten Druckbedingungen einen geringeren Siedepunkt als Anilin haben. Im Hinblick auf das Siedeverhalten gehört natürlich auch Wasser zu den Leichtsiedern; weil dieses aber keine auf Nebenreaktionen zurückzuführende organische Verunreinigung, sondern entweder ein Koppelprodukt der Anilin-Herstellung ist (Hydrierung von Nitrobenzol, Ammonolyse von Phenol) oder mit den eingesetzten Reagenzien eingeführt wird (Reduktion von Nitrobenzol mit unedlen Metallen wie Eisen in Gegenwart von Salzsäure, Ammonolyse mit wässrigem Ammoniak), wird es im Allgemeinen und auch im Rahmen der vorliegenden Erfindung separat aufgeführt. Typische Leichtsieder sind Cyclohexylamin, Cyclohexanon, Cyclohexanol und Benzol. Entsprechend werden unter *"Hochsiedern"* im Rahmen der vorliegenden Erfindung solche organischen Verbindungen (und gegebenenfalls Azeotrope) verstanden, die unter den für eine Destillation gewählten Bedingungen einen höheren Siedepunkt als Anilin haben. Unter den Bedingungen der erfindungsgemäßen Destillation gemäß Schritt b) (Kopfdruck maximal 500 mbar_{(abs.);} Anilin im Überschuss) fällt Phenol als hochsiedendes Azeotrop mit Anilin an. Weitere typische Hochsieder sind Aminophenole, Toluidine, Phenylendiamin, Diphenylamin, N-Cyclohexylanilin oder auch nicht umgesetztes Ausgangsmaterial aus der Herstellung, z. B. Nitrobenzol.

Da gängige Anilin-Herstellverfahren zunächst ein Produkt liefern, das im Allgemeinen neben Phenol (und gegebenenfalls anderen Hochsiedern) mindestens noch Leichtsieder und Wasser enthält, ist es bevorzugt, dass Schritt a) eine Destillation zur weitestgehenden Abreicherung der Leichtsieder und des Wassers umfasst. Die in Schritt a) bereitzustellende Rohanilinfraktion wird dabei als Sumpfprodukt dieser Destillation erhalten. Die Destillation wird so betrieben, dass Wasser möglichst vollständig abgetrennt wird, sodass die als Sumpfprodukt anfallende Rohanilinfraktion, bezogen auf ihre Gesamtmasse, Wasser (bestimmt per Karl-Fischer-Titration) im Bereich von 0,00 Massen-% bis 0,50 Massen-%, bevorzugt 0,00 Massen-% bis 0,20 Massen-%, besonders bevorzugt 0,00 Massen-% bis 0,10 Massen-% und außerordentlich ganz bevorzugt 0,00 Massen-% bis 0,05 Massen-%, insbesondere 0,01 Massen-% bis 0,05 Massen %, enthält.

In einer bevorzugten Ausgestaltung des Schrittes a) des erfindungsgemäßen Verfahrens erfolgt diese Destillation in einer Rektifikationskolonne, die vorzugsweise bei Unterdruck oder Umgebungsdruck, besonders bevorzugt bei Unterdruck, betrieben werden kann. Die Rektifikationskolonne kann mit allem dem Fachmann bekannten Einrichtungen zur Erhöhung der Trennleistung (wie insbesondere Schüttungen, Packungen, Böden und dergleichen) ausgestattet sein. Der Zulaufstrom kann, muss jedoch nicht zwingend, vorgewärmt werden. Besonders bevorzugt kommt eine Rektifikationskolonne mit Seitenabzug zum Einsatz, wobei die Leichtsieder über Kopf destilliert werden. Nach Kondensation und Phasentrennung wird die wässrige Phase als Rücklauf auf die Kolonne gegeben, so dass letztlich ein Anilin-Wasser-Azeotrop als Seitenstrom entnommen wird. Am Sumpf der Rektifikationskolonne fällt ein getrocknetes und an Leichtsiedern abgereichertes Anilin an.

Die eigentliche Herstellung des zu reinigenden Anilins erfolgt bevorzugt durch katalytische Hydrierung von Nitrobenzol. Das dabei erhaltene rohe Verfahrensprodukt enthält neben Anilin und Nebenkomponenten beträchtliche Anteile an Wasser als Koppelprodukt der Hydrierung (Reaktionswasser) und wird daher einer Phasentrennung unterworfen. Die dabei erhaltene wässrige Phase enthält den Hauptteil des Reaktionswassers (bis auf eine Restmenge, die infolge einer geringen Löslichkeit von Wasser in Anilin nicht durch Phasentrennung abgetrennt werden kann) und wird abgetrennt. Die verbleibende organische Phase enthält neben Anilin noch Leichtsieder und gelöstes Wasser. Typische Leichtsieder sind Cyclohexylamin, Cyclohexanon und Benzol, wobei Benzol im Allgemeinen den Hauptteil der Leichtsieder ausmacht. Im Allgemeinen enthält die in der Phasentrennung erhaltene organische Phase, bezogen auf ihre Gesamtmasse, maximal 2,00 Massen-% Benzol (d. h. 0,00 Massen-% bis 2,00 Massen-%, analog für die anderen Werte) bevorzugt maximal 1,00 Massen-% Benzol, besonders bevorzugt maximal 0,50 Massen-% Benzol.

Grundsätzlich kann diese organische Phase als Rohanilinfraktion in Schritt a) eingesetzt werden. Wie bereits erwähnt, ist es jedoch bevorzugt, diese organische Phase einer Destillation zur Abreicherung von Leichtsiedern und Wasser zu unterziehen. Die als Sumpfprodukt dieser Destillation erhaltene Rohanilinfraktion enthält, bezogen auf ihre Gesamtmasse, insbesondere maximal 0,300 Massen-% gegenüber Anilin leichter siedende organische Verbindungen (sog. Leichtsieder), bevorzugt maximal 0,200 Massen-% Leichtsieder und besonders bevorzugt maximal 0,100 Massen-% Leichtsieder. Insbesondere gilt, dass der auf die Gesamtmasse der Rohanilinfraktion bezogene Gehalt an Benzol bevorzugt maximal 0,100 Massen-%, besonders bevorzugt maximal 0,010 Massen-% und ganz besonders bevorzugt maximal 0,001 Massen-% Benzol beträgt.

Die in Schritt a) bereitgestellte, insbesondere als Sumpfprodukt einer Destillation gewonnene, Rohanilinfraktion wird in **Schritt b)** in zwei oder mehr, bevorzugt in zwei oder drei, insbesondere bevorzugt in genau zwei, Teilströme aufgeteilt.

In **Schritt c)** werden die in Schritt b) erhaltenen Teilströme der Rohanilinfraktion destilliert. Die Destillation eines jeden Teilstroms erfolgt kontinuierlich. Die Anzahl der Destillationskolonnen entspricht der Anzahl der Teilströme. Daher werden in Schritt b) zwei oder mehr, bevorzugt zwei oder drei, insbesondere bevorzugt genau zwei, Destillationskolonnen eingesetzt. Unabhängig von der Anzahl der Destillationskolonnen wird erfindungsgemäß keine der in Schritt c) eingesetzten Destillationskolonnen bei einem Kopfdruck oberhalb von 500 mbar_{(abs.)}, bevorzugt oberhalb von 400 mbar_{(abs.)}, besonders bevorzugt oberhalb von 350 mbar_{(abs.)}, betrieben. Hierdurch wird gewährleistet, dass sich Phenol als Hochsieder im Sumpfstrom der Destillationskolonnen anreichert und gereinigtes Anilin über Kopf abgezogen werden kann. Die Druckuntergrenze beträgt 1,0 mbar_{(abs.)}, bevorzugt 10 mbar_{(abs.)}, besonders bevorzugt 50 mbar_{(abs)}. Hierdurch wird bewirkt, dass der Durchmesser der eingesetzten Destillationskolonnen nicht übermäßig groß gewählt werden muss. Ferner wird durch die Begrenzung des Drucks nach unten der Aufwand in der Kondensation des zunächst gasförmig anfallenden Destillats in vertretbaren Grenzen gehalten, und die Vermeidung einer zu niedrigen Kondensationstemperatur ermöglicht eine sinnvolle Nutzung der freiwerdenden Wärme.

In den folgenden Absätzen wird die Erfindung anhand der am stärksten bevorzugten Ausführungsform mit *genau zwei* Destillationskolonnen erläutert. Ausgehend von dieser Darstellung ist es dem Fachmann leicht möglich, die Erfindung auf Anordnungen mit mehr als zwei Destillationskolonnen zu übertragen. (Die zuvor genannten technischen Randbedingungen bezüglich des maximalen Kopfdrucks der Kolonnen bleiben auch für Anordnungen mit mehr als zwei Kolonnen unverändert, und es wird jeweils die Kondensationswärme aus dem Brüdenstrom einer Kolonne genutzt, um die Kolonne mit dem nächst niedrigeren Kopfdruck zu beheizen. Auf diese Weise schlägt also in der Energiebilanz für die Reinigung nur der Wärmebedarf der ersten Kolonne zu buche. Es ist darauf zu achten, dass der Druckunterschied zwischen den Kolonnen groß genug gewählt wird, so dass sich ein hinreichender Temperaturunterschied zwischen der Kondensationstemperatur der Brüden der einen Kolonne und der Verdampfungstemperatur des Sumpfs der nächsten Kolonne ergibt. Naturgemäß lassen sich deshalb nicht beliebig viele Kolonnen miteinander koppeln. Einerseits ist der zur Verfügung stehende Druckbereich limitiert und andererseits nimmt auch die durch eine weitere Kolonne zusätzlich mögliche Energieeinsparung mit der Anzahl der Kolonnen ab.)

In den nun folgenden Ausführungen zur Ausführungsform mit zwei Destillationskolonnen wird die bei höherem Kopfdruck betriebene Destillationskolonne als *"erste Destillationskolonne"* bezeichnet, die bei niedrigerem Kopfdruck betriebene Destillationskolonne als *"zweite Destillationskolonne* ".

Sowohl in der ersten als auch in der zweiten Destillationskolonne wird Anilin gasförmig als Kopfprodukt entnommen und anschließend kondensiert. Ein Teil des dabei erhaltenen Kondensats wird als Rücklaufstrom in die Ausgangs-Destillationskolonne und/oder die jeweils andere Destillationskolonne, vorzugsweise in die Ausgangs-Destillationskolonne, zurückgeführt; der nicht zurückgeführte Anteil ist das gewünschte Produkt der entsprechenden Destillationskolonne, also das Produkt des erfindungsgemäßen Verfahrens. Ebenso wird sowohl in der ersten als auch in der zweiten Destillationskolonne ein flüssiger, an Phenol angereicherter Sumpfstrom erhalten. Ein erster Teil dieses Sumpfstromes wird jeweils zumindest teilweise verdampft und zumindest teilweise in die jeweilige Destillationskolonne zurückgeführt, während ein zweiter Teil des Sumpfstromes kontinuierlich oder diskontinuierlich ausgeschleust (das heißt dem Verfahren entzogen) wird. Bevorzugt liegt derjenige Anteil des ersten Teilstroms, der in die Destillationskolonne zurückgeführt wird, gasförmig (gegebenenfalls bis auf geringe Anteile mitgerissener Tropfen) vor.

Zur Ausführung des erfindungsgemäßen Verfahrens ist es vorteilhaft, wenn die zweite Destillationskolonne bei einem Kopfdruck im Bereich von 50 mbar_{(abs.)} bis 300 mbar_{(abs.)}, bevorzugt im Bereich von 80 mbar_{(abs.)} bis 200 mbar_{(abs.)}, besonders bevorzugt im Bereich von 100 mbar_{(abs.)} bis 150 mbar_{(abs.)} betrieben wird. Dieser Druckbereich ist so gewählt, dass einerseits die Brüden (hier also das gasförmig anfallende gereinigte Anilin; gilt für alle Destillationskolonnen des Schrittes c)) eine Kondensations-Temperatur aufweisen, die eine sinnvolle Nutzung der bei der Kondensation freiwerdenden Wärme ermöglicht. Andererseits nimmt bei höherem Druck die Trennleistung der Destillationskolonne bezogen auf das Gemisch Phenol/Anilin ab, so dass ein höheres Rücklaufverhältnis und damit ein höherer Energieeintrag erforderlich ist, um die Trennaufgabe zu bewerkstelligen.

Die erste Destillationskolonne wird bevorzugt bei einem Kopfdruck im Bereich von 250 mbar_{(abs.)} bis 500 mbar_{(abs.)}, besonders bevorzugt im Bereich von 270 mbar_{(abs.)} bis 400 mbar_{(abs.)}, ganz besonders bevorzugt im Bereich von 280 mbar_{(abs.)} bis 350 mbar_{(abs.)} betrieben. Dabei ist es besonders vorteilhaft wenn der Kopfdruck der ersten Destillationskolonne um 80 mbar_{(abs.)} bis 300 mbar_{(abs.)}, bevorzugt um 100 mbar_{(abs.)} bis 200 mbar_{(abs.)}, besonders bevorzugt um 110 mbar_{(abs.)} bis 170 mbar_{(abs.)}, höher ist als der Kopfdruck der zweiten Destillationskolonne. Auf diese Weise wird sichergestellt, dass die Kondensation der Brüden auf einem Temperaturniveau stattfindet, dass sich oberhalb der Sumpftemperatur der zweiten Destillationskolonne befindet und somit die freiwerdende Kondensationswärme für die Beheizung des Sumpfs der zweiten Destillationskolonne verwendet werden kann.

Vorteilhafterweise beträgt der Anteil der in Schritt a) bereitgestellten Rohanilinfraktion, welcher in der ersten Destillationskolonne destilliert wird, 5,00 % bis 49,00 %, bevorzugt 15,00 % bis 45,00 %, besonders bevorzugt 25,00 % bis 40,00 % der in Schritt a) bereitgestellten Rohanilinfraktion, und der verbleibende Anteil wird in der zweiten Destillationskolonne destilliert. Überraschend hat sich herausgestellt, dass sich mit dieser Aufteilung im Allgemeinen die bestmögliche Energieeffizienz erzielen lässt, das heißt, dass die Kondensationswärme aus den Brüden der ersten Destillationskolonne gerade ausreicht, um die erforderliche Energie für die Beheizung des Sumpfs der zweiten Destillationskolonne zur Verfügung zu stellen. Wird der ersten Destillationskolonne ein geringerer Anteil der Rohanilinfraktion zugeführt, so muss im Allgemeinen die zweite Destillationskolonne zusätzlich auf andere Weise, beispielsweise mittels Dampf aus anderen Prozessen, beheizt werden. Wird der ersten Destillationskolonne ein größerer Anteil der Rohanilinfraktion zugeführt, so steht im Allgemeinen aus der Brüdenkondensation mehr Wärme zur Verfügung als für die Beheizung des Sumpfes der zweiten Destillationskolonne benötigt wird. Dieser Überschuss an Wärme muss dann, sofern es nicht andere Verwendungsmöglichkeiten gibt, zum Beispiel als Abwärme über einen Luftkühler an die Atmosphäre abgegeben werden.

In einer weiteren bevorzugten Ausführungsform des erfindungsgemäßen Verfahrens wird die zweite Destillationskolonne mit einem niedrigeren Rücklaufverhältnis betrieben als die erste Destillationskolonne, das heißt, das Verhältnis von Rücklaufstrom zu Produktstrom ist in der zweiten Destillationskolonne geringer als in der ersten Destillationskolonne. Auch diese Ausführungsform führt letztlich zu einer Energie-optimierten Fahrweise. Im für das Verfahren relevanten Druckbereich vergrößert sich mit sinkendem Druck der Unterschied zwischen den Siedetemperaturen von Phenol und Anilin, so dass sich auch die Trennleistung der Destillationskolonnen bei geringerem Druck verbessert und letztlich ein geringeres Rücklaufverhältnis ausreicht, um die angestrebte Phenolspezifikation im Reinanilin zu erfüllen. Als Rücklaufmedium kann für jede der Destillationskolonnen ein beliebiges kondensiertes Anilin-Destillat aus einer der Destillationskolonnen oder eine beliebige Mischung der kondensierten Destillate verwendet werden, sofern es die Phenolspezifikation für das gereinigte Anilin erfüllt.

In einer bevorzugten Ausführungsform handelt es sich bei der ersten Destillationskolonne um eine Packungskolonne mit einem Verstärker- und einem Abtriebsteil, wobei der Verstärkerteil bevorzugt 5 bis 30, besonders bevorzugt 10 bis 25 und ganz besonders bevorzugt 15 bis 20 theoretischen Trennstufen aufweist. Der Abtriebsteil weist bevorzugt 20 bis 60, besonders bevorzugt 25 bis 40 und ganz besonders bevorzugt 28 bis 37 theoretische Trennstufen auf. Über eine Rohrleitung, die vorzugsweise ein Regelventil enthält, kann in einer bevorzugten Ausführungsform der Erfindung ein Teilstrom der Brüden der ersten Destillationskolonne dem Kondensator der Brüden der zweiten Destillationskolonne zugeführt werden, das heißt, ein Teil des der ersten Destillationskolonne gasförmig entnommenen gereinigten Anilins wird gemeinsam mit dem der zweiten Destillationskolonne gasförmig entnommenen gereinigten Anilin in einem Kondensator kondensiert. Auf diese Weise lässt sich einerseits der Kopfdruck in der ersten Destillationskolonne regeln und andererseits wird vermieden, dass sich unter den vorherrschenden Bedingungen von Druck und Temperatur nicht kondensierbare Gasanteile (zum Beispiel Ammoniak) im System anreichern.

In einer ebenfalls bevorzugten Ausführungsform handelt es sich bei der zweiten Destillationskolonne um eine Packungskolonne mit einem Verstärker- und einem Abtriebsteil, wobei der Verstärkerteil bevorzugt 5 bis 30, bevorzugt 10 bis 25 und ganz besonders bevorzugt 15 bis 20 theoretische Trennstufen aufweist. Der Abtriebsteil weist bevorzugt 20 bis 60, besonders bevorzugt 25 bis 40 und 28 bis 37 theoretische Trennstufen auf. Die Anilinbrüden werden gasförmig am Kopf der Kolonne entnommen und in mindestens einem Kondensator (der als Kaskade mehrerer Kondensatoren ausgestaltet sein kann) niedergeschlagen. Unter den vorherrschenden Bedingungen von Druck und Temperatur nicht kondensierbare Anteile werden als Abgas aus dem System abgeführt und zum Beispiel in einer thermischen Abluftreinigung behandelt.

In **Schritt d)** des erfindungsgemäßen Verfahrens wird die bei der Kondensation des Anilin-Destillats aus der ersten Destillationskolonne freiwerdende Wärme zur Bereitstellung der zur Verdampfung des ersten Teils des Sumpfstromes der zweiten Destillationskolonne erforderlichen Wärme verwendet (*Wärmeintegration*). Es ist dabei nicht zwingend erforderlich, das gesamte gasförmige Kopfprodukt der ersten Destillationskolonne für diese Wärmeintegration einzusetzen. In dem Fall, dass nicht sämtliches gasförmiges Kopfprodukt für die Durchführung der Wärmeintegration benötigt wird, kann ein Teil davon einfach kondensiert werden, und zwar vorzugsweise, indem er in die Kondensation des gasförmigen Kopfprodukts der zweiten Destillationskolonne gefahren und so zum stofflichen Bestandteil derselben wird.

Es ist möglich, den Wärmeaustausch aus den Brüden der ersten Destillationskolonne zur Beheizung der zweiten Destillationskolonne indirekt (d. h. mittels eines zusätzlichen Wärmeträgermediums) durchzuführen, indem zunächst die Kondensationswärme genutzt wird, um Wasserdampf zu erzeugen und diesen dann wiederum zur Beheizung der zweiten Destillationskolonne zu nutzen. Bevorzugt ist es jedoch, den Wärmeaustausch direkt (d. h. ohne ein zusätzliches Wärmeträgermedium) durchzuführen. Dies erfolgt erfindungsgemäß mittels einem oder mehrerer externer Verdampfer, wie z. B. Umlaufverdampfer.

In einer bevorzugten Ausführungsform der Erfindung erfolgt die Wärmeintegration gemäß Schritt d) demnach durch Verwendung eines Wärmeaustauschers, in dem sowohl das der ersten Destillationskolonne gasförmig entnommene Anilin-Destillat kondensiert als auch der erste Teil des Sumpfstromes der zweiten Destillationskolonne verdampft wird, das heißt, Kondensation und Verdampfung erfolgen in ein und demselben Wärmeaustauscher. Der Wärmeaustauscher erfüllt also die Funktion eines *Verdampfer-Kondensators.* Aufgrund der geringen Temperaturunterschiede ist es besonders bevorzugt, das zu verdampfende Sumpfprodukt mittels einer Pumpe als Zwangsumlauf durch den Wärmeaustauscher zu fördern. Besonders bevorzugt werden Rohrbündelwärmeaustauscher verwendet, wobei die Kondensation zweckmäßigerweise auf der Mantelseite erfolgt und die Verdampfung auf der Rohrseite des Wärmeaustauschers.

Der als Verdampfer-Kondensator eingesetzte Wärmeaustauscher beheizt auf diese Weise die zweite Destillationskolonne durch Übertragung der Kondensationswärme der Brüden aus der ersten Destillationskolonne auf den Sumpf der zweiten Destillationskolonne unter teilweiser Verdampfung desselben.

In Weiterbildung der Erfindung können die Brüden der zweiten Destillationskolonne in einem **Schritt e**) zumindest teilweise genutzt werden, um während ihrer Kondensation unter Nutzung der freiwerdenden Kondensationswärme (insbesondere niedrig gespannten) Dampf zu erzeugen. Diese Brüden können auch benutzt werden, um eine Trennkolonne, in welcher ein Anilin-Wasser-Azeotrop aus einem Anilin-haltigen Abwasser in einem thermischen Trennverfahren entfernt wird (Abwasser-Trennkolonne), zu beheizen. Dies kann entweder durch direkte Wärmeübertragung oder durch indirekte Wärmeübertragung mittels zuvor erzeugten Dampfes geschehen; bevorzugt ist die direkte Wärmeübertragung.

Bei dem Anilin-haltigen Abwasser handelt es sich bevorzugt um sogenanntes Prozesswasser, das bei der Hydrierung von Nitrobenzol als weiteres Produkt neben Anilin anfällt und bevorzugt durch eine Phasentrennung bei einer Temperatur im Bereich von 20 °C bis 45 °C vom Hauptteil des organischen Rohprodukts abgetrennt wird. Das Anilin-Wasser-Azeotrop aus der Abwasser-Trennkolonne (dessen Anilin-Gehalt gegenüber dem des Ausgangs-Abwassers deutlich erhöht ist) wird kondensiert und dann bevorzugt einer Phasentrennung unterworfen. Besonders bevorzugt erfolgt diese Phasentrennung zusammen mit dem in einer Hydrierung von Nitrobenzol zu Anilin anfallenden Rohprodukt aus Wasser und Anilin. Gegebenenfalls vorhandener Ammoniak kann bei geschickter Wahl der Kondensationsbedingungen gasförmig zusammen mit anderen Inert-Gasen einer Abluftreinigung zugeführt werden.

Soll die Kondensationswärme der Brüden der zweiten Destillationskolonne zur Beheizung einer Abwasser-Trennkolonne eingesetzt werden, so kann dies indirekt geschehen, indem zunächst Dampf erzeugt und dieser zur Beheizung eingesetzt wird. Auf diese Weise erzeugter Dampf kann selbstverständlich auch zur Beheizung beliebiger anderer Apparate oder zur Dampfstrippung verwendet werden.

Bevorzugt ist jedoch die direkte Verwendung der Brüden der zweiten Destillationskolonne zur Beheizung einer solchen Abwasser-Trennkolonne. Hierzu kommt wie in der bevorzugten Ausführungsform von Schritt d) ein als Verdampfer-Kondensator fungierender Wärmeaustauscher zum Einsatz, der die Kondensationswärme auf den Sumpf der Abwasser-Trennkolonne überträgt. Der Verdampfer-Kondensator ist bevorzugt als Rohrbündel-Wärmeaustauscher ausgelegt, und die Kondensation der Brüden erfolgt bevorzugt auf der Mantelseite des Apparats, während das Wasser aus dem Sumpf der Abwasser-Trennkolonne mittels einer Pumpe auf einen Verteiler gefördert wird, der es auf die Innenseite der Rohre verteilt. Gegebenenfalls wird, sofern aus den Brüden der zweiten Destillationskolonne mehr Wärme zur Verfügung steht als für die Beheizung der Abwasser-Trennkolonne erforderlich ist, ein Teilstrom der Brüden der zweiten Destillationskolonne in einem Luftkühler kondensiert. Reichen die Brüden der zweiten Destillationskolonne nicht aus, um die für die Beheizung der Abwasser-Trennkolonne erforderliche Wärme zur Verfügung zu stellen, so kann dies durch eine zusätzliche Dampfbeheizung ausgeglichen werden. Der Dampf kann entweder direkt in den Sumpf der Abwasser-Trennkolonne geleitet werden oder zur Beheizung eines zweiten Umlaufverdampfers am Sumpf der Abwasser-Trennkolonne genutzt werden. Auf diese Weise sind auch instationäre Zustände wie z. B. während des Anfahrens des Destillationssystems darstellbar ohne Gefahr zu laufen, mit Anilin belastetes Abwasser zu erzeugen.

Als Abwasser-Trennkolonne kann beispielsweise eine Packungskolonne verwendet werden, die bevorzugt 10 bis 50 theoretische Trennstufen und besonders bevorzugt 20 bis 30 theoretische Trennstufen aufweist. Aufgrund des gegenüber Wasser niedrigeren Siedepunkts des Anilin-Wasser-Azeotrops fällt das gereinigte Wasser am Sumpf der Packungskolonne an. Der Kopfdruck der Abwasser-Trennkolonne liegt bevorzugt im Bereich von 0,80 bar_{(abs.)} bis 1,20 bar_{(abs.)}, besonders bevorzugt im Bereich von 1,00 bar_{(abs.)} bis 1,10 bar_{(abs)}.

### Beispiele

In den folgenden Beispielen wird die Reinigung der bereitgestellten Rohanilinfraktion beschrieben. Es handelt sich um Modellberechnungen die mittels Aspen Plus® V7.3 erstellt wurden. Für die bereitgestellte Rohanilin-Fraktion wurde jeweils die nachfolgend aufgeführte Zusammensetzung angenommen (Gehaltsangaben sind auf die Gesamtmasse bezogene Massenanteile). Als Vergleichsbeispiele wurde die Destillation jeweils als einfache Vakuumdestillation bei einem Kopfdruck von 120 mbar_{(abs.)} bzw. 300 mbar_{(abs.)} gerechnet. Als erfindungsgemäßes Beispiel wurde die Destillation mit genau zwei Kolonnen gerechnet. In allen Fällen wurden die Rücklaufverhältnisse so angepasst, dass das Destillat jeder Kolonne einen Phenolgehalt von 50 ppm aufwies.
Anilin: 99,76 %
Wasser: 100 ppm
Weitere Leichtsieder: 220 ppm
Phenol: 700 ppm
Schwersieder: 1350 ppm

### Beispiel 1 (Vergleichsbeispiel)

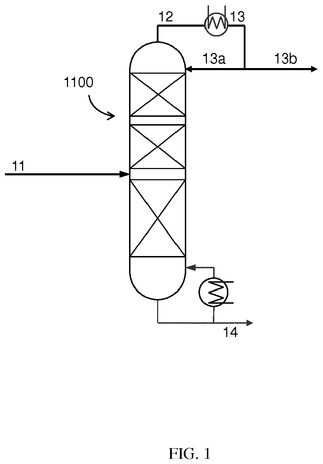

Eine Anordnung gemäß FIG. 1 wurde als Modell berechnet. Für die Kolonne wurde ein Verstärkerteil mit 18 theoretischen Stufen angenommen und ein Abtriebsteil mit 12 theoretischen Stufen. Der Kopfdruck der Kolonne (**1100**) wurde auf 120 mbar_{(abs.)} festgelegt, der Druckverlust über die Kolonne auf 45 mbar. Eine Rohanilin-Fraktion (**11**) mit oben genannter Zusammensetzung wurde mit einer Temperatur von 125 °C zwischen dem Abtriebsteil und dem Verstärkerteil der Kolonne aufgegeben. Der am Kopf der Kolonne austretende Brüdenstrom (**12**) hatte eine Temperatur von 117 °C und wurde durch Kühlung auf 110 °C vollständig kondensiert (**13**). Ein Teil dieses kondensierten Stroms (**13**) wurde als Rücklauf (**13a**) am Kopf der Kolonne aufgegeben und ein weiterer Teil wurde als Produkt (**13b**) abgenommen. Das Verhältnis aus Rücklauf- und Produktstrom (R/E-Verhältnis; **13a/13b**) wurde so angepasst, dass der Phenolgehalt im Produktstrom (**13b**) 50 ppm bezogen auf den gesamten Strom betrug. Dieser Gehalt konnte mit einem R/E-Verhältnis von 1,175 erreicht werden. Am Sumpf der Kolonne wurde ein Strom (**14**) enthaltend 94 % Anilin, 4 % Schwersieder und 2 % Phenol jeweils bezogen auf den gesamten Strom ausgeschleust und es stellte sich eine Temperatur von 126 °C ein. Zur Beheizung des Kolonnensumpfs wurde eine erforderliche spezifische Heizleistung von 311 KW/t_{Rohanilin} ermittelt, die Kondensation der Brüden erforderte eine spezifische Kühlleistung von 320 kW/t_{Rohanilin}

### Beispiel 2 (Vergleichsbeispiel)

Die Simulation erfolgte analog zu Beispiel 1, mit dem Unterschied, dass der Kopfdruck der Kolonne (**1100**) auf 300 mbar_{(abs.)} festgesetzt wurde (Kopftemperatur 143 °C). Die Sumpftemperatur stellte sich zu 148 °C ein. Das erforderliche R/E-Verhältnis zum Erreichen eines Phenolgehalts von 50 ppm im Produktstrom (**13b**) betrug nun 2,48. Die erforderliche spezifische Heizleistung für die Destillation betrug 544 KW/t_{Rohanilin} und zur Kondensation der Brüden (12) wurden 552 KW/t_{Rohanilin} benötigt. Die ermittelte Produktzusammensetzung entsprach exakt der aus Beispiel 1.

### Beispiel 3 (erfindungsgemäß)

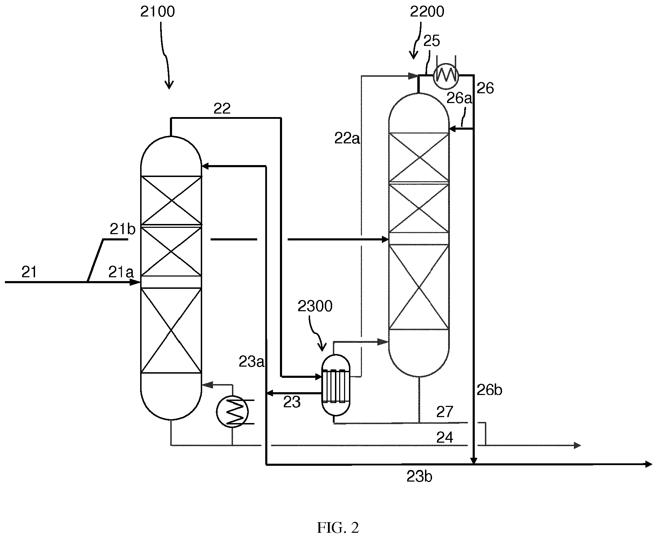

Eine Anordnung gemäß FIG. 2 wurde als Modell berechnet. Für beide Kolonnen (**2100, 2200**) wurde jeweils ein Verstärkerteil mit 18 theoretischen Stufen angenommen und ein Abtriebsteil mit 12 theoretischen Stufen. Der Kopfdruck der ersten Kolonne (**2100**) wurde auf 300 mbar_{(abs.)} festgelegt, der Druckverlust über die Kolonne auf 45 mbar. Der Kopfdruck der zweiten Kolonne (**2200**) wurde auf 120 mbar_{(abs.)} festgelegt und der Druckverlust über die Kolonne wiederum auf 45 mbar. Eine Rohanilin-Fraktion (**21**) mit oben genannter Zusammensetzung wurde in zwei Teilströme im Verhältnis 64 : 36 aufgeteilt, wobei der kleinere Strom **(21a)** zwischen den Abtriebs- und Verstärkerteil der ersten Kolonne (**2100**) aufgegeben wurde und der größere Strom (**21b**) zwischen den Abtriebs- und Verstärkerteil der zweiten Kolonne **(2200).** Am Sumpf der Kolonne wurde jeweils ein Strom (**24, 27**) enthaltend 94 % Anilin, 4 % Schwersieder und 2 % Phenol, jeweils bezogen auf den gesamten Strom, ausgeschleust, und es stellte sich in der ersten Kolonne **(2100)** eine Sumpftemperatur von 148 °C sowie in der zweiten Kolonne **(2200)** eine Sumpftemperatur von 126 °C ein. Es wurde für jede der Kolonnen **(2100, 2200)** das R/E-Verhältnis angepasst, so dass jeweils ein Produktstrom **(23b, 26b)** mit einem Phenolgehalt von 50 ppm, bezogen auf den gesamten Produktstrom, erzeugt wurde. Die Zusammensetzung des Produktstroms war wiederum die gleiche wie in den Beispielen 1 und 2. In der ersten Kolonne war hierzu ein R/E-Verhältnis von 2,94 erforderlich, in der zweiten Kolonne ein R/E-Verhälntis von 1,24. Die Kopftemperaturen der beiden Kolonnen betrugen 143 °C für die erste Kolonne und 117 °C für die zweite Kolonne.

Die Brüden **(22)** der ersten Kolonne **(2100)** konnten bei einer Kondensationstemperatur von 141 °C zu 99,85 % kondensiert werden. Damit fiel die Kondensationswärme fast vollständig auf einem Temperaturniveau an, das es erlaubt, die Wärme zur Beheizung der zweiten Kolonne (**2200**) zu nutzen, deren Sumpftemperatur mit 126 °C um 15 K unterhalb der Kondensationstemperatur der ersten Kolonne liegt. Die in der Gasphase verbleibenden Anteile wurden zusätzlich in den Kondensationsapparat der zweiten Kolonne geleitet und dort niedergeschlagen.

Für die Kondensation der Brüden **(25)** aus der zweiten Kolonne **(2200)** zuzüglich des nicht kondensierten Anteils **(22a)** aus der ersten Kolonne **(2100),** welcher den Wärmeaustauscher **(2300),** der zum Beheizen des Sumpfes der zweiten Kolonne **(2200)** diente, verlässt, war eine spezifische Kühlleistung von 201 KW/t_{Rohanilin} erforderlich. Die Brüden konnten im Modell bei einer Temperatur von knapp 114 °C vollständig kondensiert werden. Diese Kondensationstemperatur liegt damit 14 K oberhalb der Siedetemperatur von Wasser bei Atmosphärendruck, so dass die Energie genutzt werden kann, um z. B. Wasser bei Atmosphärendruck zu verdampfen, was also eine weitere Energie-Integration ermöglicht.

Zur Energiebilanz lässt sich festhalten, dass zur Beheizung der ersten Kolonne **(2100)** eine spezifische Heizleistung von 201 KW/t_{Rohanilin} erforderlich war. Die spezifische Heizleistung der zweiten Kolonne **(2200)** betrug 196 KW/t_{Rohanilin}. Diese wurde vollständig durch die Kondensation der Brüden aus der ersten Kolonne zur Verfügung gestellt, welche eine spezifische Kühlleistung in der gleichen Höhe erfordert. In Summe konnte also durch das erfindungsgemäße Verfahren die erforderliche spezifische Heizleistung um ca. ein Drittel reduziert werden.

## Patentansprüche

1. Verfahren zur Reindarstellung von Anilin, umfassend die folgenden Schritte:
a) Bereitstellen einer Rohanilinfraktion enthaltend, bezogen auf die Gesamtmasse von Anilin und Phenol in der Rohanilinfraktion, 80,000 Massen-% bis 99,999 Massen-% Anilin und 0,001 Massen-% bis 20,000 Massen-% Phenol;
b) Aufteilen der Rohanilinfraktion aus Schritt a) in einen ersten Teilstrom und einen zweiten Teilstrom;
c) Destillieren des ersten Teilstroms aus Schritt b) in einer ersten Destillationskolonne und Destillieren des zweiten Teilstroms aus Schritt b) in einer zweiten Destillationskolonne, wobei die erste Destillationskolonne bei einem höheren Kopfdruck betrieben wird als die zweite Destillationskolonne unter der Maßgabe, dass die erste Destillationskolonne und die zweite Destillationskolonne jeweils bei einem Kopfdruck im Bereich von 1,0 mbar_{(abs.)} bis 500 mbar_{(abs.)} betrieben werden, und wobei der ersten Destillationskolonne und der zweiten Destillationskolonne jeweils
• ein Anilin-Destillat gasförmig als Kopfprodukt entnommen und anschließend kondensiert wird, wobei ein erster Teilstrom des kondensierten Anilin-Destillats als Rücklaufstrom in die erste Destillationskolonne und/oder in die zweite Destillationskolonne zurückgeführt und ein zweiter Teilstrom des kondensierten Anilin-Destillats als Produktstrom abgeführt wird, und
• ein an Phenol angereicherter flüssiger Sumpfstrom entnommen wird, von dem ein erster Teil partiell oder vollständig verdampft und in die jeweilige Destillationskolonne zurückgeführt wird, und von dem ein zweiter Teil ausgeschleust wird;
d) Verwenden der bei der Kondensation des Anilin-Destillats aus der ersten Destillationskolonne freiwerdenden Wärme zur Bereitstellung der zur Verdampfung des ersten Teils des Sumpfstromes der zweiten Destillationskolonne erforderlichen Wärme.

2. Verfahren gemäß Anspruch 1, bei welchem das Bereitstellen der Rohanilinfraktion in Schritt a) die Destillation einer neben Phenol zusätzlich Wasser und Leichtsieder enthaltenden Anilinfraktion umfasst, wobei die in Schritt a) bereitzustellende Rohanilinfraktion als Sumpfprodukt dieser Destillation erhalten wird und, bezogen auf ihre Gesamtmasse, maximal 0,50 Massen-% Wasser enthält.

3. Verfahren gemäß Anspruch 2, bei welchem die neben Phenol zusätzlich Wasser und Leichtsieder enthaltende Anilinfraktion durch katalytische Hydrierung von Nitrobenzol, gefolgt von einer Phasentrennung in eine organische und eine wässrige Phase, als organische Phase erhalten wird.

4. Verfahren gemäß einem der vorstehenden Ansprüche, bei welchem die Rohanilinfraktion in Schritt b) in genau zwei Teilströme aufgeteilt wird, welche in Schritt c) in genau zwei Destillationskolonnen destilliert werden.

5. Verfahren gemäß Anspruch 4, bei welchem die zweite Destillationskolonne bei einem Kopfdruck im Bereich von 50 mbar_{(abs.)} bis 300 mbar_{(abs.)} betrieben wird.

6. Verfahren gemäß einem der Ansprüche 4 oder 5, bei welchem die erste Destillationskolonne bei einem Kopfdruck im Bereich von 250 mbar_{(abs.)} bis 500 mbar_{(abs.)} betrieben wird.

7. Verfahren gemäß einem der Ansprüche 4 bis 6, bei welchem die erste Destillationskolonne bei einem Kopfdruck betrieben wird, der um 80 mbar_{(abs.)} bis 300 mbar_{(abs.)} höher ist als der Kopfdruck der zweiten Destillationskolonne.

8. Verfahren gemäß einem der Ansprüche 4 bis 7, bei welchem mindestens ein Teil der Kondensation des Anilin-Destillats aus der ersten Destillationskolonne und die Verdampfung des ersten Teils des Sumpfstromes der ersten Destillationskolonne in ein und demselben Wärmeaustauscher durchgeführt werden.

9. Verfahren gemäß einem der Ansprüche 4 bis 8, bei welchem der Anteil der in Schritt a) bereitgestellten Rohanilinfraktion, welcher in der ersten Destillationskolonne destilliert wird, 5,00 % bis 49,00 % der in Schritt a) bereitgestellten Rohanilinfraktion beträgt und der verbleibende Anteil in der zweiten Destillationskolonne destilliert wird.

10. Verfahren gemäß einem der Ansprüche 4 bis 9, bei welchem das Verhältnis von Rücklaufstrom zu Produktstrom in der zweiten Destillationskolonne geringer ist als in der ersten Destillationskolonne.

11. Verfahren gemäß einem der Ansprüche 4 bis 10, bei welchem ein Teil des der ersten Destillationskolonne gasförmig entnommenen Anilin-Destillats gemeinsam mit dem der zweiten Destillationskolonne gasförmig entnommenen gereinigten Anilin in einem Kondensator kondensiert wird.

12. Verfahren gemäß einem der vorstehenden Ansprüche, das den weiteren Schritt umfasst:
e) Verwenden der bei der Kondensation des der zweiten Destillationskolonne gasförmig entnommenen Anilin-Destillats anfallenden Wärme zur Erzeugung von Dampf und/oder zur Beheizung einer Trennkolonne, in welcher ein Anilin-Wasser-Azeotrop aus einem Anilin-haltigen Abwasser entfernt wird.

13. Verfahren gemäß Anspruch 12, bei welchem die Trennkolonne bei einem Kopfdruck im Bereich von 0,80 bar_{(abs.)} bis 1,20 bar_{(abs.)} betrieben wird.

14. Verfahren gemäß einem der vorstehenden Ansprüche, bei welchem die in Schritt a) bereitgestellte Rohanilinfraktion, bezogen auf die Gesamtmasse von Anilin und Phenol in der Rohanilinfraktion, 95,000 Massen-% bis 99,995 Massen-% Anilin und 0,005 Massen-% bis 5,000 Massen-% Phenol enthält.

15. Verfahren nach einem der vorstehenden Ansprüche, bei welchem der erste Teilstrom des kondensierten Anilin-Destillats jeder Destillationskolonne als Rücklaufstrom in diejenige Destillationskolonne zurückgeführt wird, welcher das Anilin-Destillat entnommen wurde.

## Claims

1. Process for the preparation of pure aniline, comprising the following steps:
a) providing a crude aniline fraction containing, with respect to the total weight of aniline and phenol in the crude aniline fraction, 80.000 % by weight to 99.999 % by weight of aniline and 0.001 % by weight to 20.000 % by weight of phenol;
b) dividing the crude aniline fraction from step a) into a first partial stream and a second partial stream;
c) distilling the first partial stream from step b) in a first distillation column and distilling the second partial stream from step b) in a second distillation column, wherein the first distillation column is operated at a higher head pressure than the second distillation column, with the proviso that the first and the second distillation columns are respectively operated at a head pressure in the range from 1.0 mbar_{(abs.)} to 500 mbar_{(abs.)}, and wherein from the first and the second distillation columns respectively
• a gaseous aniline distillate is removed as the overhead product and subsequently condensed, wherein a first partial stream of the condensed aniline distillate is returned to the first and/or the second distillation column as a recycle stream and a second partial stream of the condensed aniline distillate is removed as the product stream, and
• a liquid bottom stream which is concentrated in phenol is removed, from which a first portion is partially or completely vaporised and is returned to the respective distillation column, and from which a second portion is discharged;
d) using the heat released during the condensation of the aniline distillate from the first distillation column to provide the heat necessary for vaporising the first portion of the bottom stream from the second distillation column.

2. Process according to Claim 1, in which the provision of the crude aniline fraction in step a) comprises distilling an aniline fraction which additionally contains water and low boilers in addition to phenol, wherein the crude aniline fraction to be provided in step a) is obtained as a bottom product from this distillation and, with respect to its total weight, contains a maximum of 0.50 % by weight of water.

3. Process according to Claim 2, in which the aniline fraction additionally containing water and low boilers in addition to phenol is obtained as an organic phase by the catalytic hydrogenation of nitrobenzene, followed by a phase separation into the organic phase and an aqueous phase.

4. Process according to one of the preceding claims, in which the crude aniline fraction in step b) is divided into exactly two partial streams which are distilled in step c) in exactly two distillation columns.

5. Process according to Claim 4, in which the second distillation column is operated at a head pressure in the range from 50 mbar_{(abs.)} to 300 mbar_{(abs.)}.

6. Process according to either of Claims 4 and 5, in which the first distillation column is operated at a head pressure in the range from 250 mbar_{(abs.)} to 500 mbar_{(abs.)}.

7. Process according to one of Claims 4 to 6, in which the first distillation column is operated at a head pressure which is 80 mbar_{(abs.)} to 300 mbar_{(abs.)} higher than the head pressure of the second distillation column.

8. Process according to one of Claims 4 to 7, in which at least part of the condensation of the aniline distillate from the first distillation column and the vaporisation of the first portion of the bottom stream from the first distillation column are carried out in one and the same heat exchanger.

9. Process according to one of Claims 4 to 8, in which the proportion of the crude aniline fraction provided in step a) which is distilled in the first distillation column is 5.00 % to 49.00 % of the crude aniline fraction provided in step a), and the remaining proportion is distilled in the second distillation column.

10. Process according to one of Claims 4 to 9, in which the ratio of the recycle stream to the product stream in the second distillation column is lower than in the first distillation column.

11. Process according to one of Claims 4 to 10, in which a portion of the gaseous aniline distillate removed from the first distillation column is condensed in a condenser together with the gaseous purified aniline removed from the second distillation column.

12. Process according to one of the preceding claims, which comprises the further step:
e) using the heat generated during the condensation of the gaseous aniline distillate removed from the second distillation column for the production of steam and/or for heating a separation column in which an aniline-water azeotrope is removed from an aniline-containing effluent.

13. Process according to Claim 12, in which the separation column is operated at a head pressure in the range from 0.80 bar_{(abs.)} to 1.20 bar_{(abs.)}.

14. Process according to one of the preceding claims, in which the crude aniline fraction provided in step a), with respect to the total weight of aniline and phenol in the crude aniline fraction, contains 95.000 % by weight to 99.995 % by weight of aniline and 0.005 % by weight to 5.000 % by weight of phenol.

15. Process according to one of the preceding claims, in which the first partial stream of the condensed aniline distillate of each distillation column is returned as a recycle stream to that distillation column from which the aniline distillate was removed.

## Revendications

1. Procédé pour la préparation d'aniline pure, comprenant les étapes suivantes :
a) mise à disposition d'une fraction d'aniline brute contenant, par rapport à la masse totale d'aniline et de phénol dans la fraction d'aniline brute, 80,000% en masse à 99,999% en masse d'aniline et 0,001% en masse à 20,000% en masse de phénol ;
b) répartition de la fraction d'aniline brute de l'étape a) en un premier flux partiel et un deuxième flux partiel ;
c) distillation du premier flux partiel de l'étape b) dans une première colonne de distillation et distillation du deuxième flux partiel de l'étape b) dans une deuxième colonne de distillation, la première colonne de distillation fonctionnant à une pression de tête supérieure à celle de la deuxième colonne de distillation, sous réserve que la première colonne de distillation et la deuxième colonne de distillation fonctionnent à chaque fois à une pression de tête de 1,0 mbar_{(abs.)} à 500 mbars_{(abs.)} et, à chaque fois,
• un distillat d'aniline étant prélevé sous forme gazeuse en tant que produit de tête de la première colonne de distillation et de la deuxième colonne de distillation et ensuite condensé, un premier flux partiel du distillat condensé d'aniline étant recyclé comme flux de reflux dans la première colonne de distillation et/ou dans la deuxième colonne de distillation et un deuxième flux partiel du distillat condensé d'aniline étant évacué comme flux produit et
• un flux de fond liquide enrichi en phénol étant prélevé de la première colonne de distillation et de la deuxième colonne de distillation, dont une première partie est partiellement ou totalement évaporée et recyclée dans la colonne de distillation respective et dont une deuxième partie est soutirée ;
d) utilisation de la chaleur libérée lors de la condensation du distillat d'aniline de la première colonne de distillation pour la mise à disposition de la chaleur nécessaire pour l'évaporation de la première partie du flux de fond de la deuxième colonne de distillation.

2. Procédé selon la revendication 1, dans lequel la mise à disposition de la fraction d'aniline brute dans l'étape a) comprend la distillation d'une fraction d'aniline contenant, outre du phénol, en plus de l'eau et des substances à bas point d'ébullition, la fraction d'aniline brute à mettre à disposition dans l'étape a) étant obtenue comme produit de fond de cette distillation et contenant, par rapport à sa masse totale, au maximum 0,50% en masse d'eau.

3. Procédé selon la revendication 2, dans lequel la fraction d'aniline contenant, outre du phénol, en plus de l'eau et des substances à bas point d'ébullition, est obtenue par hydrogénation catalytique de nitrobenzène, suivie d'une séparation de phases en une phase organique et en une phase aqueuse, sous forme de phase organique.

4. Procédé selon l'une quelconque des revendications précédentes, dans lequel la fraction d'aniline brute dans l'étape b) est répartie en exactement deux flux partiels, qui sont distillés dans l'étape c) dans exactement deux colonnes de distillation.

5. Procédé selon la revendication 4, dans lequel la deuxième colonne de distillation fonctionne à une pression de tête dans la plage de 50 mbars_{(abs.)} à 300 mbars_{(abs.)}.

6. Procédé selon l'une quelconque des revendications 4 ou 5, dans lequel la première colonne de distillation fonctionne à une pression de tête dans la plage de 250 mbars_{(abs.)} à 500 mbars_{(abs.)}.

7. Procédé selon l'une quelconque des revendications 4 à 6, dans lequel la première colonne de distillation fonctionne à une pression de tête qui est supérieure de 80 mbars_{(abs.)} à 300 mbars_{(abs.)} à la pression de tête de la deuxième colonne de distillation.

8. Procédé selon l'une quelconque des revendications 4 à 7, dans lequel au moins une partie de la condensation du distillat d'aniline provenant de la première colonne de distillation et l'évaporation de la première partie du flux de fond de la première colonne de distillation sont réalisées dans un seul et même échangeur de chaleur.

9. Procédé selon l'une quelconque des revendications 4 à 8, dans lequel la proportion de la fraction d'aniline brute mise à disposition dans l'étape a), qui est distillée dans la première colonne de distillation, représente 5,00% à 49,00% de la fraction d'aniline brute mise à disposition dans l'étape a) et la proportion restante est distillée dans la deuxième colonne de distillation.

10. Procédé selon l'une quelconque des revendications 4 à 9, dans lequel le rapport du flux de reflux au flux produit dans la deuxième colonne de distillation est inférieur à celui dans la première colonne de distillation.

11. Procédé selon l'une quelconque des revendications 4 à 10, dans lequel une partie du distillat d'aniline prélevé sous forme gazeuse de la première colonne de distillation est condensée conjointement avec l'aniline purifiée prélevée sous forme gazeuse de la deuxième colonne de distillation dans un condenseur.

12. Procédé selon l'une quelconque des revendications précédentes, dans lequel l'autre étape comprend :
e) l'utilisation de la chaleur obtenue lors de la condensation du distillat d'aniline prélevé sous forme gazeuse de la deuxième colonne de distillation pour la production de vapeur et/ou pour le chauffage d'une colonne de séparation, dans laquelle un azéotrope aniline-eau est éliminé d'une eau résiduaire contenant de l'aniline.

13. Procédé selon la revendication 12, dans lequel la colonne de séparation fonctionne à une pression de tête dans la plage de 0,80 bar_{(abs.)} à 1,20 bar_{(abs.)}.

14. Procédé selon l'une quelconque des revendications précédentes, dans lequel la fraction d'aniline brute mise à disposition dans l'étape a) contient, par rapport à la masse totale d'aniline et de phénol dans la fraction d'aniline brute, 95,000% en masse à 99,995% en masse d'aniline et 0,005% en masse à 5,000% en masse de phénol.

15. Procédé selon l'une quelconque des revendications précédentes, dans lequel le premier flux partiel du distillat condensé d'aniline de chaque colonne de distillation est recyclé sous forme de flux de reflux dans la colonne de distillation dont le distillat d'aniline a été prélevé.
